# FASCICULE DE BREVET EUROPEEN

(11) **EP 3 887 809 B1**
(45) Date de publication et mention de la délivrance du brevet: **19.04.2023**
(21) Numéro de dépôt: 19809074.8
(22) Date de dépôt: 26.11.2019
(51) Int. Cl.: G01N 24/10

(54) **MÉTHODE DE DÉTERMINATION DE LA SENSIBILITÉ À L'OXYDATION D'UN LIANT BITUMINEUX PAR RÉSONANCE PARAMAGNÉTIQUE ÉLECTRONIQUE**
VERFAHREN ZUR BESTIMMUNG DER OXIDATIONSEMPFINDLICHKEIT EINES BITUMINÖSEN BINDEMITTELS MITTELS ELEKTRONENSPINRESONANZ
METHOD FOR DETERMINING THE SENSITIVITY TO OXIDATION OF A BITUMINOUS BINDER BY ELECTRON SPIN RESONANCE

(30) Priorité: 29.11.2018 FR 1872084
(43) Date de publication de la demande: 06.10.2021
(73) Titulaire: TotalEnergies OneTech, 92400 Courbevoie (FR); Centre national de la recherche scientifique, 75016 Paris (FR); Université de Lille, 59800 Lille (FR)
(72) Inventeur: MERCE, Manuel, 40230 BÉNESSE-MAREMNE (FR); PONDAVEN, Simon, 69007 LYON (FR); MARCHAND, Philippe, 69360 COMMUNAY (FR); VEZIN, Hervé, 59650 VILLENEUVE D'ASCQ (FR); BEN TAYEB MEZIANE, Karima, 59493 VILLENEUVE D'ASCQ (FR)
(74) Mandataire: Lavoix
(86) Numéro de dépôt international: PCT/EP2019/082585
(87) Numéro de publication internationale: WO 2020/109303

(56) Documents cités:
- DATABASE WPI Week 197737 Thomson Scientific, London, GB; AN 1977-66438Y XP002794130, -& SU 529 410 A (PECHENYI B G) 14 février 1977 (1977-02-14)
- CESARE OLIVIERO ROSSI ET AL: "Effects of Natural Antioxidant Agents on the Bitumen Aging Process: An EPR and Rheological Investigation", APPLIED SCIENCES, vol. 8, no. 8, 19 août 2018 (2018-08-19), page 1405, XP055620187, DOI: 10.3390/app8081405 cité dans la demande
- H. MASMOUDI ET AL: "Spectroscopic study of bituminous oxidative stress", SPECTROCHIMICA ACTA. PART A: MOLECULAR AND BIOMOLECULAR SPECTROSCOPY, vol. 60, no. 6, mai 2004 (2004-05), pages 1343-1348, XP055620780, NL ISSN: 1386-1425, DOI: 10.1016/j.saa.2003.10.032

## Description

La présente invention concerne l'analyse de la stabilité au vieillissement, notamment par sa sensibilité à l'oxydation, d'un liant bitumineux par résonance paramagnétique électronique. La présente invention concerne également une méthode de classification de liants bitumineux en fonction de leur sensibilité à l'oxydation, une méthode de suivi en continu de la production d'un liant bitumineux et une méthode de contrôle de la qualité d'un liant bitumineux.

Le vieillissement d'un liant bitumineux est notamment dû à l'existence de mécanismes d'oxydation. Ce vieillissement progressif cause des changements dans la structure du liant bitumineux, et une altération de ses propriétés macroscopiques, le rendant par exemple plus fragile et donc plus facilement fissurable.

Il y a donc un intérêt à pouvoir évaluer la propension d'un liant bitumineux à résister à son vieillissement, notamment à résister au phénomène d'oxydation.

Buckmaster et al. (Advances in Chemistry Séries, 1993, 229 (Magnetic Resonance of Carbonaceous Solids), 483-506) décrit la détermination par RPE de paramètres spectroscopiques de charbons bitumeux ayant subi une oxydation à basse température. Rossi et al. (Applied Sciences, 2018, 8, 1405-1419) décrit l'étude par RPE de l'influence de l'ajout d'antioxydants naturels dans des bitumes afin de prévenir leur vieillissement.

Cependant, ces méthodes ne concernent pas l'analyse d'un liant bitumineux brut ou ne permettent pas de mesurer l'impact d'un vieillissement sur le long terme.

Il existe donc un intérêt à développer une méthode d'analyse d'un liant bitumineux permettant de manière rapide et fiable de déterminer sa stabilité au vieillissement, notamment par sa sensibilité à l'oxydation.

Un objectif de la présente invention est de fournir une méthode d'analyse de la stabilité au vieillissement d'un liant bitumineux notamment par sa sensibilité à l'oxydation, qui soit rapide et fiable.

Un autre objectif de la présente invention est de fournir une méthode permettant de classifier un liant bitumineux en fonction de sa stabilité au vieillissement, et notamment par sa sensibilité à l'oxydation.

La présente invention concerne donc une méthode d'analyse de la stabilité au vieillissement, notamment par la sensibilité à l'oxydation, d'un liant bitumineux comprenant :
a) l'analyse par résonance paramagnétique électronique d'un échantillon du liant bitumineux et la mesure de l'intégrale du signal des radicaux stables carbo-centrés ;
b) le vieillissement accéléré du liant bitumineux comprenant: i) le chauffage dudit liant bitumineux, suivi du ii) chauffage sous pression du liant bitumineux obtenu à l'issu de l'étape i) ;
c) l'analyse par résonance paramagnétique électronique d'un échantillon du liant bitumineux vieilli obtenu à l'étape b) et la mesure de l'intégrale du signal des radicaux stables carbo-centrés ;
d) la comparaison de l'intégrale du signal des radicaux stables carbo-centrés du liant bitumineux obtenue à l'étape a) et celle obtenu à l'étape c).

Plus la valeur obtenue à l'étape d) est importante plus le liant bitumineux est sensible à l'oxydation et donc au vieillissement.

Dans le cadre de la présente invention, on considère que les termes « liant bitumineux » et « bitume » sont synonymes.

Parmi les bitumes selon l'invention, on peut citer tout d'abord les bitumes d'origine naturelle, ceux contenus dans des gisements de bitume naturel, d'asphalte naturel ou les sables bitumineux et les bitumes provenant du raffinage du pétrole brut.

Les bitumes selon l'invention sont avantageusement choisis parmi les bitumes provenant du raffinage du pétrole brut. Les bitumes peuvent être choisis parmi les bitumes ou mélanges de bitumes provenant du raffinage du pétrole brut, en particulier des bitumes contenant des asphaltènes ou des brais.

Les bitumes peuvent être obtenus par des procédés conventionnels de fabrication des bitumes en raffinerie, en particulier par distillation directe et/ou distillation sous vide du pétrole. Ces bitumes peuvent être éventuellement viscoréduits et/ou désasphaltés et/ou rectifiés à l'air de manière connue par l'homme du métier. Il est courant de procéder à la distillation sous vide des résidus atmosphériques provenant de la distillation atmosphérique de pétrole brut. Ce procédé de fabrication correspond, par conséquent, à la succession d'une distillation atmosphérique et d'une distillation sous vide, la charge alimentant la distillation sous vide correspondant aux résidus atmosphériques. Ces résidus sous vide issus de la tour de distillation sous vide peuvent également constituer un bitume selon l'invention. Il est également courant d'injecter de l'air dans une charge composée habituellement de distillats et de produits lourds provenant de la distillation sous vide de résidus atmosphériques provenant de la distillation du pétrole. Ce procédé permet d'obtenir une base soufflée, ou semi-soufflée ou oxydée ou rectifiée à l'air ou rectifiée partiellement à l'air.

Les différents bitumes ou bases bitumes obtenus par les procédés de raffinage peuvent être combinés entre eux pour obtenir le meilleur compromis technique. Le bitume peut aussi être un bitume de recyclage. Les bitumes peuvent être des bitumes de grade dur ou de grade mou.

Avantageusement, le bitume est choisi parmi les bitumes d'origine naturelle, parmi les bitumes issus du raffinage du pétrole brut tels que les résidus de distillation atmosphérique, les résidus de distillation sous vide, les résidus viscoréduits, les résidus soufflés, leurs mélanges et leurs combinaisons

Dans la méthode selon l'invention, le liant bitumineux est analysé tel qu'obtenu en sortie de raffinerie. De préférence, le liant bitumineux ne contient aucun additif, comme par exemple des antioxydants. De préférence, le liant bitumineux n'a subi aucune étape d'extraction par un solvant organique ou un mélange d'au moins deux solvants organiques, comme par exemple les hydrocarbures aliphatiques ou aromatiques. De manière préférentielle, le liant bitumineux est analysé dans son intégralité.

Parmi les méthodes d'analyses connues, la résonance paramagnétique électronique (RPE) est particulièrement adaptée, car elle est relativement rapide et ne nécessite que quelques milligrammes ou dizaines de milligrammes d'échantillon.

Il existe des méthodes d'analyse par RPE qui permettent de mesurer l'évolution de liant bitumineux ayant subi une oxydation.

Dans le cadre de la présente invention, l'analyse par RPE consiste à enregistrer le spectre RPE d'un échantillon de liant bitumineux sur une gamme de champ étroite (entre 0,34 T (3400 G) et 0,36 T (3600 G)), afin de se concentrer sur la détection du signal correspondant aux radicaux stables carbo-centrés présents dans ce liant bitumineux. Ce signal est situé de préférence entre 0,34 T (3400 G) et 0,35 T (3500 G).

Dans le cadre de la présente invention, on entend par radical stable « carbo-centré » un radical porté sur un carbone.

Dans le cadre de la présente invention, la « mesure de l'intégrale du signal des radicaux stables carbo-centrés » consiste à traiter le signal RPE correspondant aux radicaux stables carbo-centrés pour obtenir la valeur de l'intégrale de ce signal, cette valeur d'intégrale étant normalisée par la masse de l'échantillon analysé. Selon l'invention, l'intégrale du signal des radicaux libres correspond à l'aire sous le pic correspondant aux radicaux stables carbo-centrés. Le traitement du signal RPE est connu de l'homme du métier.

De préférence, la valeur de l'intégrale du signal RPE des radicaux stables carbo-centrés du liant bitumineux n'inclut pas les ions vanadyl.

De préférence, dans la méthode selon l'invention, l'étape i) du vieillissement accéléré du liant bitumineux est effectuée entre 100 °C et 200 °C pendant une durée comprise entre 5 et 200 min, de préférence entre 150 °C et 190 °C pendant une durée comprise entre 50 et 100 min, préférentiellement à 163 °C pendant 75 min.

De manière préférentielle, l'étape i) correspond à un essai de détermination de la résistance au durcissement sous l'effet de la chaleur et de l'air (ou Rolling Thin Film Over Test (RTFOT) selon la terminologie anglo-saxonne). Ce test est encadré par la norme EN 12607-1.

De préférence, dans la méthode selon l'invention, l'étape ii) du vieillissement accéléré du liant bitumineux s'effectue entre 50 °C et 150 °C, sous une pression variant de 0,5 à 5 MPa, pendant une durée comprise entre 1 et 100 heures, de préférence entre 70 °C et 120 °C sous une pression variant de 1 à 3 MPa, pendant une durée comprise entre 10 et 30 heures , préférentiellement à 100 °C, sous 2,1 MPa de pression et pendant 20 h.

De manière préférentielle, l'étape ii) correspond à un test de vieillissement long-terme accéléré réalisé dans un récipient de vieillissement sous pression (ou Pressure Ageing Vessel (PAV) selon la terminologie anglo-saxonne). Ce test est encadré par la norme EN 14769.

La présente invention concerne également une méthode selon la revendication 6 de classification d'un liant bitumineux en fonction de sa stabilité au vieillissement, notamment en fonction de sa sensibilité à l'oxydation, comprenant :
1) l'analyse par résonance paramagnétique électronique d'un échantillon du liant bitumineux et la mesure de l'intégrale du signal des radicaux stables carbo-centrés ;
2) le vieillissement accéléré du liant bitumineux ;
3) l'analyse par résonance paramagnétique électronique d'un échantillon du liant bitumineux vieilli obtenu à l'étape 2) et la mesure de l'intégrale du signal des radicaux stables carbo-centrés ;
4) la détermination d'un pourcentage d'augmentation du nombre de radicaux stables carbo-centrés dans le liant bitumineux par comparaison de l'intégrale du signal des radicaux stables carbo-centrés du liant bitumineux obtenue à l'étape 1) et celle obtenue à l'étape 3) ;
5) la détermination du niveau de sensibilité du liant bitumineux :
   faible pour un pourcentage d'augmentation du nombre de radicaux stables inférieur à 60%,
   moyen pour un pourcentage d'augmentation du nombre de radicaux stables compris entre 60% et 80%, ou
   élevé pour un pourcentage d'augmentation du nombre de radicaux stables supérieur à 80%.

Dans le cadre de la présente invention, le « vieillissement accéléré du liant bitumineux » selon la méthode de classification ci-dessus correspond : i) au chauffage du liant bitumineux, suivi du ii) chauffage sous pression du liant bitumineux obtenu à l'issu de l'étape i), les étapes i) et ii) étant telles que définies plus haut.

Dans le cadre de la présente invention, le « pourcentage d'augmentation du nombre de radicaux stables carbo-centrés dans le liant bitumineux » correspond au ratio de la valeur d'intégrale du signal RPE correspondant aux radicaux stables carbo-centrés présents dans le liant bitumineux avant et après vieillissement du liant bitumineux. La valeur d'intégrale du signal RPE est telle que définie plus haut.

De manière préférentielle, le niveau de sensibilité du liant bitumineux selon la méthode de l'invention est :
extrêmement faible pour un pourcentage d'augmentation du nombre de radicaux stables inférieur à 40%,
très faible pour un pourcentage d'augmentation du nombre de radicaux stables compris entre 40% et 50%,
faible pour un pourcentage d'augmentation du nombre de radicaux stables compris entre 50% et 60%,
moyen pour un pourcentage d'augmentation du nombre de radicaux stables compris entre 60% et 70%,
moyennement élevé pour un pourcentage d'augmentation du nombre de radicaux stables compris entre 70% et 80%,
élevé pour un pourcentage d'augmentation du nombre de radicaux stables du nombre de radicaux stables compris entre 80% et 90%, ou
très élevé pour un pourcentage d'augmentation du nombre de radicaux stables supérieur à 90%.

La présente invention concerne également une méthode de suivi en continu de la production d'un liant bitumineux en ligne dans une raffinerie, dans laquelle ledit liant bitumineux produit en continu est analysé en plusieurs endroits du procédé de production selon la méthode d'analyse définie plus haut.

De préférence, le liant bitumineux est analysé à au moins 2 endroits différents.

Cette méthode de suivi permet avantageusement d'adapter le procédé de production du liant bitumineux, afin obtenir un liant bitumineux final présentant une résistance au vieillissement, et notamment à l'oxydation, suffisante.

De préférence, on entend par un liant bitumineux final présentant une « résistance à l'oxydation suffisante » un liant bitumineux présentant un niveau de sensibilité déterminé comme étant « extrêmement faible », « très faible », « faible » ou « moyen », tel que défini dans la présente invention.

Pour ce faire, la méthode de suivi en continu selon l'invention permet notamment d'adapter la proportion des bruts pétroliers de différentes origines mis en jeu dans le procédé de raffinage duquel est issu le liant bitumineux.

La présente invention concerne également une méthode de contrôle en continu de la qualité d'un liant bitumineux lors de sa production, dans laquelle ledit liant bitumineux est analysé en sortie de procédé de production par la méthode de l'invention.

Selon un mode de réalisation préférentiel, la méthode de contrôle de la qualité d'un liant bitumineux selon l'invention permet de déterminer la nécessité d'ajouter un additif au liant bitumineux afin de le rendre plus résistant au vieillissement, notamment à l'oxydation. De préférence, la méthode de contrôle de la qualité d'un liant bitumineux indique qu'il est nécessaire d'ajouter un additif lorsque le liant bitumineux présente un niveau de sensibilité à l'oxydation « moyennement élevé », « élevé » ou « très élevé », tel que défini dans la présente invention.

De manière préférentielle, la méthode de contrôle de la qualité d'un liant bitumineux permet de caractériser des liants bitumineux qui ont une très bonne résistance au vieillissement, notamment les liants bitumineux présentant un niveau de sensibilité à l'oxydation « élevé » ou « très élevé ».

### FIGURES

[Fig 1] La Figure 1 représente un spectre RPE d'un liant bitumineux.
[Fig 2] La Figure 2 représente l'évolution en pourcentage du nombre de radicaux stables carbo-centrés après RTFOT ou RTFOT + PAV normalisés par rapport à l'échantillon non vieilli.

L'invention va maintenant être décrite grâce aux exemples suivants, non limitatifs.

### 1. Vieillissement des liants bitumineux

Le liant bitumineux subit d'abord un test RTFOT, pendant lequel un film fin de 80 g de liant bitumineux est mis en rotation continue autour de la surface interne d'une bouteille en verre à une température de 163 ± 0.5 °C. Le test dure 75 min, pendant lesquelles de l'air chaud est injecté toutes les 3-4 secondes.

Ce test est considéré comme une simulation réaliste des conditions subies par un liant bitumineux lors de sa mise en oeuvre, il est encadré par la norme européenne EN 12607-1.

Le liant bitumineux subit ensuite un deuxième test, le test PAV, pendant lequel 50 g de liant ayant subi le test RTFOT sont chauffés à 100 °C sous 2,1 MPa de pression pendant 20 h.

Ce test permet de simuler le vieillissement sur une période de 7 à 10 ans d'un liant bitumineux en service, et est encadré par la norme EN 14769.

### 2. Préparation des échantillons à analyser par RPE

Avant analyse par RPE, les liants bitumineux vieillis et non vieillis sont stockés à - 20 °C dans des récipients en aluminium. Un échantillon est prélevé à environ 3 mm de profondeur grâce à une lame pointue, et modelé à la main pour obtenir un échantillon de la forme d'un grain de riz d'environ 30 mg, 1 cm de long et 3 mm de diamètre. L'échantillon est analysé à 25 °C.

### 3. Analyse des échantillons par RPE

Les analyses ont été faites en mode onde continue (ou continuous wave (CW) selon la terminologie anglo-saxonne) sur un spectromètre Bruker ELEXSYS-E500. Les paramètres RPE utilisés sont repris dans le tableau 1.

### [Table 1]

**Tableau 1. Paramètres RPE utilisés pour l'analyse d'échantillons de liant bitumineux**

| | |
|---|---|
| Fréquence microondes | 9.3 ± 0.1 GHz |
| Puissance de microondes | 0.25 mW |
| Centre du champ | 3500 G |
| Amplitude du champ | 150.0 G |
| Modulation d'amplitude | 2 G |
| Fréquence de modulation | 100 KHz |
| Constante de temps | 40.96 ms |
| Temps d'acquisition | 60 s |

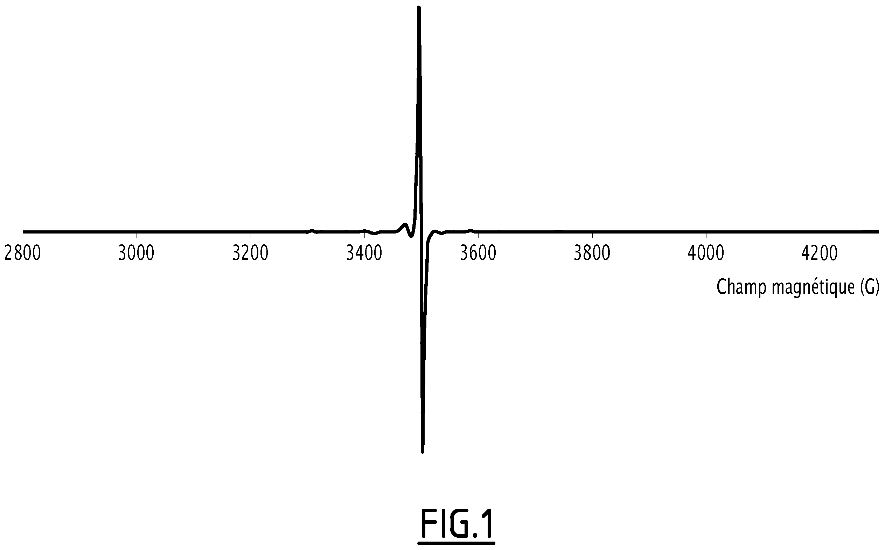

Un spectre RPE typique d'un échantillon de liant bitumineux est représenté figure 1. Le signal majoritaire correspond aux radicaux stables carbo-centrés, et les signaux minoritaires de part et d'autre du signal majoritaire correspondent aux ions vanadyl.

### 4. Résultats

Neuf échantillons de liant bitumineux ont été analysés selon la méthode de l'invention.

Il s'agit de liants bitumineux de différentes origines géographiques (les bruts sont Iraniens, Vénézuéliens, de l'Oural, Arabe léger & lourd etc.), tous issus du raffinage du pétrole mais obtenus par des procédés de raffinages différents :
- Y0820-18 : bitume 35/50 de la raffinerie de Feyzin - procédé de raffinage classique ;
- X0490-06 : bitume 35/50 de la raffinerie de Feyzin - procédé de raffinage classique ;
- Y0625-05 : bitume 35/50 de la raffinerie de Donges - procédé de raffinage classique ;
- 16-00-756-10 : bitume 35/50 de la raffinerie de Feyzin - procédé de raffinage classique ;
- X1029-03 ; X1030-05 et X1031-02 : 30/45, bitumes d'origine Cubaine dont le procédé de raffinage n'est pas connu ;
- 35/50 : correspond à un bitume 35/50 de la raff de Feyzin, procédé classique ;
- 70/100 : correspond à un bitume de grade 70/100 de la raffinerie de Feyzin, procédé d'obtention classique.

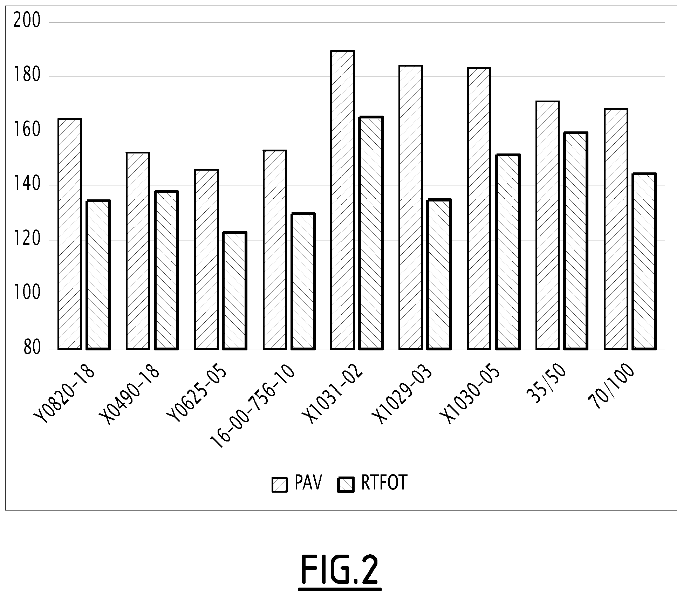

Les spectres RPE de ces échantillons ont été enregistrés avant vieillissement, après le test RTFOT (étape i) selon l'invention) puis après les tests RTFOT + PAV (étapes i) et ii) selon l'invention). La figure 2 représente l'évolution en pourcentage du nombre de radicaux stables carbo-centrés après chaque étape de vieillissement, normalisé par rapport à l'échantillon non vieilli.

Les tests RTFOT et RTFOT + PAV résultent tous les deux en une augmentation du nombre de radicaux stables carbo-centrés. En considérant que plus cette augmentation est importante, plus le liant bitumineux est sensible à l'oxydation, et donc au vieillissement, il est possible d'établir un classement des 9 liants bitumineux testés en fonction de leur résistance au vieillissement. Cependant, ce classement est différent selon que le liant bitumineux a subi seulement le test RTFOT, ou bien le test RTFOT + PAV.

Le test RTFOT simule les conditions subies par un liant bitumineux lors de sa mise en oeuvre, alors que le test PAV permet de simuler le vieillissement sur une période de 7 à 10 ans d'un liant bitumineux en service.

Ainsi, les résultats obtenus sur les bitumes ayant subi le test RTFOT + PAV permettent d'analyser de manière fiable la stabilité au vieillissement sur le long terme d'un liant bitumineux, alors qu'une méthode de vieillissement impliquant seulement un chauffage du liant bitumineux pendant une certaine durée (comme le test RTFOT), ne permet pas d'analyser de manière rapide et fiable la stabilité au vieillissement d'un liant bitumineux.

## Revendications

1. Méthode d'analyse de la stabilité au vieillissement, notamment par la sensibilité à l'oxydation d'un liant bitumineux comprenant :
a) l'analyse par résonance paramagnétique électronique d'un échantillon du liant bitumineux et la mesure de l'intégrale du signal des radicaux stables carbo-centrés ;
b) le vieillissement accéléré du liant bitumineux comprenant: i) le chauffage dudit liant bitumineux, suivi du ii) chauffage sous pression du liant bitumineux obtenu à l'issu de l'étape i) ;
c) l'analyse par résonance paramagnétique électronique d'un échantillon du liant bitumineux vieilli obtenu à l'étape b) et la mesure de l'intégrale du signal des radicaux stables carbo-centrés ;
d) la comparaison de l'intégrale du signal des radicaux stables carbo-centrés du liant bitumineux obtenue à l'étape a) et celle obtenue à l'étape c).

2. Méthode selon la revendication 1, dans laquelle l'étape i) du vieillissement accéléré du liant bitumineux s'effectue entre 100 °C et 200 °C pendant une durée comprise entre 5 et 200 min, de préférence entre 150 °C et 190 °C pendant une durée comprise entre 50 et 100 min, préférentiellement à 163 °C pendant 75 min.

3. Méthode selon la revendication 1 ou 2, dans laquelle l'étape ii) du vieillissement accéléré du liant bitumineux s'effectue entre 50 °C et 150 °C, sous une pression variant de 0,5 à 5 MPa, pendant une durée comprise entre 1 et 100 heures, de préférence entre 70 °C et 120 °C sous une pression variant de 1 à 3 MPa, pendant une durée comprise entre 10 et 30 heures, préférentiellement à 100 °C, sous 2,1 MPa de pression et pendant 20 h.

4. Méthode selon l'une quelconque des revendications précédentes, dans laquelle l'étape i) du vieillissement accéléré du liant bitumineux correspond à un essai de détermination de la résistance au durcissement sous l'effet de la chaleur et de l'air.

5. Méthode selon l'une quelconque des revendications précédentes, dans laquelle l'étape ii) du vieillissement accéléré du liant bitumineux correspond à un test de vieillissement long-terme accéléré réalisé dans un récipient de vieillissement sous pression.

6. Méthode de classification d'un liant bitumineux en fonction de sa stabilité au vieillissement, notamment en fonction de sa sensibilité à l'oxydation, comprenant :
une méthode d'analyse de la stabilité au vieillissement selon l'une quelconque des revendications 1 à 5 avec une détermination d'un pourcentage d'augmentation du nombre de radicaux stables carbo-centrés dans le liant bitumineux par l'étape d), et une
détermination du niveau de sensibilité du liant bitumineux :
faible pour un pourcentage d'augmentation du nombre de radicaux stables inférieur à 60%,
moyen pour un pourcentage d'augmentation du nombre de radicaux stables compris entre 60% et 80%, ou
élevé pour un pourcentage d'augmentation du nombre de radicaux stables supérieur à 80%.

7. Méthode selon la revendication 6, dans laquelle le niveau de sensibilité du liant bitumineux est :
extrêmement faible pour un pourcentage d'augmentation du nombre de radicaux stables inférieur à 40%,
très faible pour un pourcentage d'augmentation du nombre de radicaux stables compris entre 40% et 50%,
faible pour un pourcentage d'augmentation du nombre de radicaux stables compris entre 50% et 60%,
moyen pour un pourcentage d'augmentation du nombre de radicaux stables compris entre 60% et 70%,
moyennement élevé pour un pourcentage d'augmentation du nombre de radicaux stables compris entre 70% et 80%,
élevé pour un pourcentage d'augmentation du nombre de radicaux stables du nombre de radicaux stables compris entre 80% et 90%, ou
très élevé pour un pourcentage d'augmentation du nombre de radicaux stables supérieur à 90%.

8. Méthode de suivi en continu de la production d'un liant bitumineux, ladite méthode étant utilisée en ligne dans une raffinerie, dans laquelle ledit liant bitumineux produit en continu est régulièrement analysé selon la méthode de classification définie selon la revendication 6 ou 7.

9. Méthode de contrôle de la qualité d'un liant bitumineux, dans laquelle ledit liant bitumineux est analysé en sortie de procédé de production par la méthode de classification définie selon la revendication 6 ou 7.

## Patentansprüche

1. Analyseverfahren der Alterungsstabilität, insbesondere durch die Oxidationsempfindlichkeit eines bituminösen Bindemittels, umfassend:
a) Analysieren einer Probe des bituminösen Bindemittels mittels Elektronenspinresonanz und Messen des Integrals des Signals der stabilen carbo-zentrischen Radikale;
b) beschleunigtes Altern des bituminösen Bindemittels, umfassend: i) Erhitzen des bituminösen Bindemittels, gefolgt von ii) Erhitzen unter Druck des aus Schritt i) erlangten bituminösen Bindemittels;
c) Analysieren einer Probe des in Schritt b) erlangten gealterten bituminösen Bindemittels mittels Elektronenspinresonanz und Messen des Integrals des Signals der stabilen carbo-zentrischen Radikale;
d) Vergleichen des Integrals des Signals der carbo-zentrischen stabilen Radikale des bituminösen Bindemittels, das in Schritt a) erlangt wird, mit dem, das in Schritt c) erlangt wird.

2. Verfahren nach Anspruch 1, wobei der Schritt i) des beschleunigten Alterns des bituminösen Bindemittels bei 100 °C bis 200 °C über eine Dauer von 5 bis 200 Min., vorzugsweise bei 150 °C bis 190 °C über eine Dauer von 50 bis 100 Min., bevorzugt bei 163 °C über 75 Min., durchgeführt wird.

3. Verfahren nach Anspruch 1 oder 2, wobei Schritt ii) des beschleunigten Alterns des bituminösen Bindemittels bei 50 °C bis 150 °C unter einem Druck von 0,5 bis 5 MPa über eine Dauer von 1 bis 100 Stunden, vorzugsweise bei 70 °C bis 120 °C unter einem Druck von 1 bis 3 MPa über eine Dauer von 10 bis 30 Stunden, vorzugsweise bei 100 °C unter einem Druck von 2,1 MPa und über 20 Stunden, durchgeführt wird.

4. Verfahren nach einem der vorherigen Ansprüche, wobei der Schritt i) des beschleunigten Alterns des bituminösen Bindemittels einem Test zum Bestimmen des Widerstands gegen Aushärtung unter Wärme- und Lufteinwirkung entspricht.

5. Verfahren nach einem der vorherigen Ansprüche, wobei der Schritt ii) des beschleunigten Alterns des bituminösen Bindemittels einem beschleunigten Langzeitalterungstest entspricht, der in einem Druckalterungsbehälter durchgeführt wird.

6. Klassifizierungsverfahren eines bituminösen Bindemittels abhängig von seiner Alterungsstabilität, insbesondere abhängigvon seiner Oxidationsempfindlichkeit, umfassend: ein Analyesverfahren der Alterungsstabilität nach einem der Ansprüche 1 bis 5 mit einer Bestimmung einer prozentualen Zunahme der Anzahl stabiler carbozentrischer Radikale in dem bituminösen Bindemittel durch Schritt d) und einer Bestimmung des Empfindlichkeitsgrads des bituminösen Bindemittels:
niedrig bei einem prozentualen Anstieg der Zahl der stabilen Radikalen um weniger als 60 %,
mittel für einen prozentualen Anstieg der Anzahl stabiler Radikale zwischen 60 % und 80 %, oder
hoch bei einem prozentualen Anstieg der Zahl der stabilen Radikalen um mehr als 80 %.

7. Verfahren nach Anspruch 6, wobei der Empfindlichkeitsgrad des bituminösen Bindemittels wie folgt ist:
extrem niedrig bei einem prozentualen Anstieg der Zahl der stabilen Radikalen um weniger als 40 %,
sehr niedrig bei einem prozentualen Anstieg der Zahl der stabilen Radikale zwischen 40 % und 50 %,
niedrig bei einem prozentualen Anstieg der Zahl der stabilen Radikale zwischen 50 % und 60 %,
durchschnittlich für einen prozentualen Anstieg der Anzahl stabiler Radikale zwischen 60 % und 70 %,
mittelhoch für einen prozentualen Anstieg der Anzahl stabiler Radikale zwischen 70 % und 80 %,
hoch für einen prozentualen Anstieg der Anzahl stabiler Radikale der Anzahl stabiler Radikale zwischen 80% und 90% oder sehr hoch für einen prozentualen Anstieg der Anzahl stabiler Radikale von über 90 %.

8. Kontinuierliches Überwachungsverfahren der Produktion eines bituminösen Bindemittels, wobei das Verfahren online in einer Raffinerie verwendet wird, in der das kontinuierlich hergestellte bituminöse Bindemittel regelmäßig gemäß dem nach Anspruch 6 oder 7 definierten Klassifizierungsverfahren analysiert wird.

9. Verfahren zur Kontrolle der Qualität eines bituminösen Bindemittels, wobei das bituminöse Bindemittel am Ausgang des Herstellungsverfahrens durch das nach Anspruch 6 oder 7 definierte Klassifizierungsverfahren analysiert wird.

## Claims

1. A method for analysing the ageing stability of a bituminous binder, in particular by reference to its susceptibility to oxidation, comprising:
a) analysing a sample of the bituminous binder by means of electron spin resonance and measuring the integral of the signal of the carbon-centred stable radicals;
b) accelerated ageing of the bituminous binder, comprising: i) heating the bituminous binder, followed by ii) heating the bituminous binder resulting from step i) under pressure;
c) analysing a sample of the aged bituminous binder obtained from step b) by means of electron spin resonance and measuring the integral of the signal of the carbon-centred stable radicals;
d) comparing the integral of the signal of the carbon-centred stable radicals of the bituminous binder obtained from step a) and that obtained from step c).

2. The method according to claim 1, wherein step i) of accelerated ageing of the bituminous binder occurs between 100 and 200 °C for a duration of between 5 and 200 min, preferably between 150 and 190 °C over a duration of between 50 and 100 min, more preferably at 163 °C for 75 min.

3. The method according to claim 1 or 2, wherein step ii) of accelerated ageing of the bituminous binder occurs between 50 and 150 °C, at a pressure ranging from 0.5 - 5 MPa, over a duration of between 1 and 100 h, preferably between 70 and 120 °C at a pressure ranging between 1 and 3 MPa, over a duration of between 10 and 30 h, more preferably at 100 °C, at a pressure of 2.1 MPa over a period of 20 h.

4. The method according to any of the preceding claims, wherein step i) of accelerated ageing of the bituminous binder corresponds to a Rolling Thin Film Over Test.

5. The method according to any of the preceding claims, wherein step ii) of accelerated ageing of the bituminous binder corresponds to a long-term accelerated ageing test in a pressure ageing vessel.

6. A method for classifying a bituminous binder as a function of its ageing stability, in particular as a function of its susceptibility to oxidation, comprising:
a method for analysing the ageing stability according to any one of claims 1 to 5 with a determination of a percent increase in the number of carbon-centred stable radicals in the bituminous binder via step d), and
determining the level of susceptibility of the bituminous binder:
low in the case of a percent increase in the number of stable radicals of less than 60 %;
medium in the case of a percent increase in the number of stable radicals between 60% and 80 %, or
high in the case of a percent increase in the number of stable radicals of more than 80%.

7. The method according to claim 6, wherein the level of susceptibility of the bituminous binder is:
extremely low in the case of a percent increase in the number of stable radicals of less than 40%;
very low in the case of a percent increase in the number of stable radicals between 40% and 50%,
low in the case of a percent increase in the number of stable radicals between 50% and 60%,
medium in the case of a percent increase in the number of stable radicals between 60% and 70%,
medium-high in the case of a percent increase in the number of stable radicals between 70% and 80%,
high in the case of a percent increase in the number of stable radicals in the number of stable radicals between 80% and 90 %, or
very high in the case of a percent increase in the number of stable radicals of more than 90%.

8. A method for continuous monitoring of the production of a bituminous binder, wherein the method is used on a production line in a refinery, wherein the continuously produced bituminous binder is regularly analysed according to the classification method according to claim 6 or 7.

9. A method for quality control of a bituminous binder, wherein the bituminous binder is analysed at the end of the production process by the classification method according to claim 6 or 7.
